# EUROPEAN PATENT APPLICATION

(11) **EP 1 053 755 A2**
(43) Date of publication of application: **22.11.2000**
(21) Application number: 00401288.6
(22) Date of filing: 11.05.2000
(51) Int. Cl.: A61L 2/26

(54) **Medical instrument sterilization system**

(30) Priority: 14.05.1999 US 312127; 14.05.1999 US 312126; 14.05.1999 US 312125
(71) Applicant: Poly Vac, Inc., Manchester, NH 03103-3300 (US)
(72) Inventor: Wood, Timothy, Weare, New Hampshire 03281 (US)
(74) Representative: Des Termes, Monique

(57) **Abstract**

A medical instrument sterilization system in one aspect includes a sterilization tray having a bottom wall (30) and an array of ventilation/mounting holes (32) in the bottom wall. The ventilation/mounting holes (32) are arranged evenly spaced at least in part in the bottom wall. Preferably the ventilation/mounting holes comprise a central portion (34) and one or more lobes (36). In a particularly preferred embodiment, the ventilation/mounting holes comprise a plurality of like cruciform-shaped holes. Also included are one or more rigid brackets (40) having posts (48) arranged to project through the ventilation/mounting holes (32) for anchoring at selected positions on the tray. In another aspect of the invention, there is provided a sterilization tray system which comprises an outer box-like case (110) having holes in the case bottom wall through which steam or other steriliant may circulate through the case. Pairs of standards (160) are affixed vertically to opposite the case side and/or end walls. A plurality of spaced holes (164,166) is provided in each standard. The holes serve as anchoring points for shelf brackets (170) or fixtures for supporting trays (126,128) at selected positions in the case. In another aspect a surgical tray sterilization tray comprises a frame (210) made of a synthetic plastic material having a metal plate (220) insert floatably mounted in the frame and forming the tray bottom or floor.

## Description

This invention relates to the field of sterilization of surgical instruments. More particularly, the invention relates to an improved system for securing surgical instruments at fixed positions in sterilization trays.

Surgical instruments are often transported in trays prior to use. The instruments are usually laid out in a certain way in the tray and subjected to sterilization in a steam autoclave or similar sterilization apparatus. In order to maintain separations between the various instruments in the tray, the instruments are supported or retained by brackets, clips, posts or other fixation devices positioned in the tray. Following sterilization, the tray full of instruments is transported to an operating room and placed close to the surgical team whose members withdraw the instruments from the tray as needed for a particular surgical procedure. Many times, the instruments are selectively arranged in the tray so that they can be picked from the tray in the general order that they are needed for the particular procedure. Examples of such trays are found in U.S. Patent Nos. 4,643,303; 5,424,048 and 5,492,671.

As seen from the above patents, the known devices for organizing and fixating medical instruments in a tray include various types of brackets, clips and posts which project up from the bottom of the tray, the instruments being held in place within slots and clip openings and/or between the posts. A plurality of such fixation devices are spaced running parallel or perpendicular to each other in the tray so that they engage and support the opposite sides or ends of various different length instruments.

Most prior art fixation devices are able to effectively locate and hold instruments which are more or less straight and regularly shaped. However, they are not particularly suitable for fixating oddly or irregularly shaped instruments such as retractors and other longer instruments that have, e.g. ring handles. This is because there is insufficient flexibility in the placement of the various fixation devices within the tray as to enable the devices to closely engage the instruments while still organizing the instruments in an efficient layout within the tray. This results from the fact that the fixation devices often are plugged into the ventilation holes usually present in the bottom of the tray such that a fixation device only can be placed where there are holes in the bottom of the tray.

As the number of such holes is limited by manufacturing cost, required tray bottom strength and the need to prevent the instruments from projecting through the holes, so also are the positions of the various fixation devices. Consequently, either the tray contains too few properly fixated instruments or a larger number of instruments some of which may not be properly fixated. Thus, if the tray is shaken or tilted, instruments may become disengaged from the fixation devices and assume skewed positions in the tray so that they may become damaged and difficult to remove without upsetting other instruments in the tray. In extreme cases, loose instruments may even fall out of the tray and become contaminated. Since a tray may contain a complete set of instruments needed for a particular surgical procedure, this may require that another full tray of sterilized instruments be made available to the surgical team.

Another consideration is that the instruments required to perform a specific surgical procedure may vary greatly between hospitals and even surgical teams within specific hospitals. Therefore, it is practically impossible to design a standard tray configuration that will be acceptable for all hospitals and surgical teams. Thus, an optimum instrument fixation arrangement is one which is enormously flexible so that it can be customized to each individual hospital and surgical team, because the numbers and types of instruments being presented in the trays change constantly.

In order to overcome the aforesaid problems of the prior art, there is proposed in U.S. Patent 5,827,487 a surgical instrument fixation device for use in a sterilization tray, comprising a rail of optional length and having at least two pegs projecting from the underside of the rail which are sized and spaced apart so as to be able to plug into at least two of the ventilation holes in the bottom wall of the tray. As disclosed in the aforesaid U.S. Patent 5,827,487, the ventilation holes are usually arranged in a rectangular array of columns and rows so that a rail can be positioned at any location within a column or row, i.e. running parallel or perpendicular. Typically, the rails are releasably fixed in position by threaded fasteners driven from the underside of the tray.

However, since surgical instruments come in a wide variety of shapes and forms, it is impractical to have a single rail type fixation device for all types of surgical instruments. Thus, the art has developed various systems wherein supports and dividers for the surgical instruments are provided in modular or kit form for selective positioning within the tray, for example, by plugging selected support elements through holes in a portion of the tray and fixing the element in place. The support elements can thus be arranged to match the shape of the surgical instrument to be sterilized.

Examples of such products are shown in U.S. Patent No. 4,135,868 to Sheinholz and U.S. Patent No. 5,384,103 to Miler. Similar products are commercially available from companies such as Poly-Vac, Incorporated of Manchester, N.H. and other suppliers. Some of these prior devices include integrally molded stubs, for example positioned on the bottom of the flexible inserts, which stubs can be locked into the ventilation holes in the tray as shown, for example, in Fig. 1 of Miller U.S. Patent No. 5,384,103. They may also comprise separate, rigid holding elements such as shown in Fig. 3 of the above '103 patent where a rigid holder for the support element is fastened by threaded fasteners to the tray or to a shelf carried by the tray. In the '868 patent, the support element for a soft sponge rubber, constituting a hold down pad, is supported by a channel member having outwardly extending buttons which can be forced into ventilation holes in the cover or base of the sterilizing tray. Another prior U.S. patent, U.S. Patent No. 4,798,292 shows hollow pegs having elongated legs which are used for attachment to a perforated sterilizer tray.

While all of the systems described in the prior patents and commercially available products provide a certain amount of flexibility, they do not provide both strong security for the support members and low cost. Nor do they allow for ease of removal of a securely mounted support so that the supports can be differently positioned in the sterilization trays for holding different shapes of surgical instruments to be sterilized.

In order to address this latter problem, there is described in U.S. Patent 5,599,512 a commercially available (from Poly Vac, Inc., of Manchester, New Hampshire) sterilization support element provided with sets of resiliently deformable bayonet type fingers for locking the support elements in position in ventilation holes in bottom wall of a sterilization tray. The support elements are simply and easily removed and repositioned in the tray by means of a simple tool which engages the ends of the bayonet fingers, and pushes them back through the holes.

While all of the systems described in the above prior patents, and in commercially available products provide a certain amount of flexibility, they all have disadvantages. They all require tools for assembling and/or removing the supports or dividers. Also, those prior art systems employing threaded fasteners, locking rings or the like, require extra parts counts. Additionally, since the mounting holes are arranged in parallel rows, standard fixation devices and separators are limited to parallel and/or perpendicular positioning relative to one another.

Originally, sterilization trays were made of metal. Metal had an advantage in that it has a relatively high thermal mass, thus leading to improved evaporation of steam or other sterilant following exposure to the steam or sterilant. However, metal is difficult to work and is heavy. Also, metal could dull or nick delicate surgical instruments. Accordingly, more recently, advances in high temperature resistant plastics have led to the commercialization of sterilization trays made of plastic. Plastic has certain advantages over metal. For one, the trays may be molded. Also, trays made of plastic weigh significantly less than trays made of metal. On the other hand, plastic has a significantly lower thermal mass than metal. Thus, trays made of plastic are not as forgiving as metal trays.

It is thus an object of the present invention to overcome the aforesaid and other disadvantages of the prior art.

More particularly, in accordance with one aspect of the present invention, a sterilization tray in which the bottom wall of a sterilization tray is populated at least in part with evenly spaced ventilation/mounting holes. Preferably the ventilation/mounting holes comprise a central hole and one or more lobes, in a particularly preferred embodiment, the ventilation/mounting holes comprise a plurality of like cruciform-shaped holes. Completing the invention are one or more brackets having posts arranged to project through the ventilation/mounting holes for anchoring at selected positions on the tray.

In accordance with another aspect of the invention, there is provided an improved sterilization tray system comprising an outer box-like case having holes in the case bottom walls through which steam or other sterilant may circulate through the case. Pairs of brackets are mounted vertically opposite one another to the case side and/or end walls. A plurality of spaced holes is provided in each bracket. The holes serve as anchoring points for shelf brackets or fixtures for supporting trays at selected positions within the case.

In yet a third aspect of the present invention a sterilization, transporting and storage container tray for surgical instruments comprises a frame made of a synthetic plastic material, and having a metal plate insert mounted in the frame and forming the tray bottom or floor. In order to accommodate different rates of thermal expansion, the metal plate insert is attached to the plastic frame by means of resiliently deformable or floating fasteners. Alternatively, the metal insert plate is floated in the frame.

Yet other objects and advantages of the present invention will be apparent in the following detailed description of the invention, taken in conjunction with the accompanying drawings wherein like numerals depict like parts, and wherein:
FIG. 1 is a top view of a sterilization tray system made in accordance with a first aspect of the present invention;
FIGS. 1a, 1b, 1c and 1d are enlarged top plan views showing details of mounting/ventilation holes in accordance with the present invention;
FIG. 2 is an enlarged perspective view of one form of bracket portion of the present invention;
FIG. 3 is an enlarged, perspective view of a mounting post portion of a bracket of the present invention;
FIGS. 4, 5 and 6 show alternative embodiments of sterilization trays made in accordance with the present invention;
FIG. 7 is a view similar to FIG. 2 showing another embodiment of fastening bracket made in accordance with the present invention;
FIG. 8 is an enlarged perspective view of an alternative fastening bracket in accordance with the present invention;
FIG. 9 is a view similar to FIG. 8, but taken from the bottom;
FIG. 10 is an enlarged view showing details of the post portion of the bracket of Fig. 7;
FIG. 11 is an enlarged view, similar to FIG. 7 and showing another alternative embodiment of fastening bracket in accordance with the present invention;
FIG. 12 is an exploded perspective view of a sterilization tray system made in accordance with a second aspect of the present invention;
FIG. 13 is a top perspective view of the sterilization tray system of FIG. 12;
FIG. 14 is a cross-sectional perspective view of a sterilization tray system of the present invention taken along III-III of FIG. 13, and showing a sterilization tray and case assembled together in accordance with the present invention;
FIG. 15 and FIG. 16 are front and back views, respectively, and showing details of a preferred form of vertical adjusting rails in accordance with the present invention;
FIG. 17 is an enlarged perspective view showing a preferred form of shelf mounting bracket in accordance with the present invention;

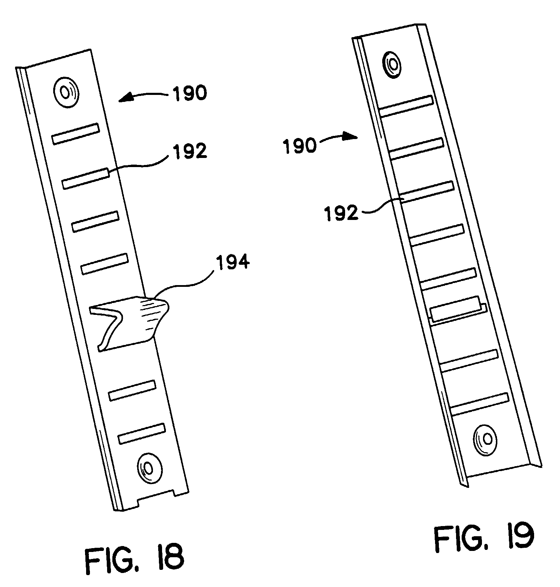

FIGS. 18 and 19 are views similar to FIGS. 15 and 16, respectively, and showing details of an alternative form of vertical adjusting rails in accordance with the present invention;
FIG. 20 is a side perspective view of a sterilization tray made in accordance with a third aspect of the present invention;
FIG. 21 is a cross-sectional view taken along lines II-II of FIG. 20;
FIG. 22 is a side elevation view of a rivet useful in accordance with the present invention;
FIG. 23 is an enlarged view of the indicated portion of FIG. 21; and
FIGS. 24 to 26 show alternative embodiments of the invention.

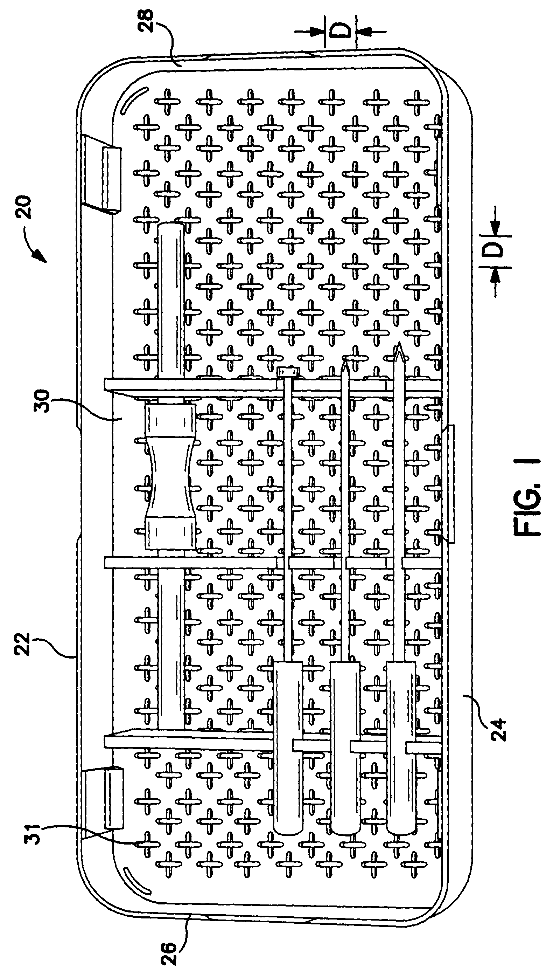

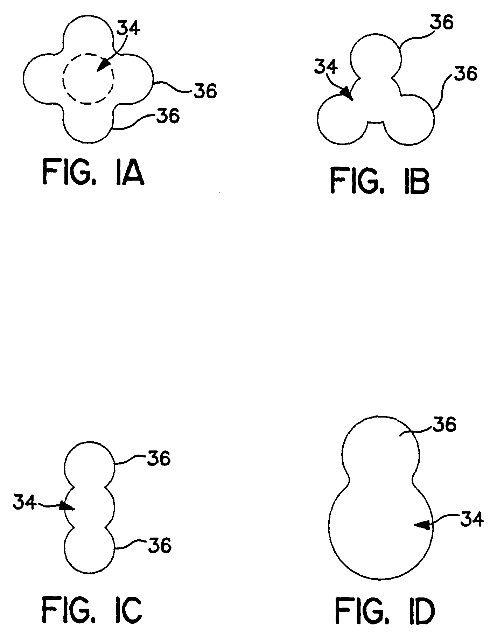

Referring now to Figs. 1-3, the sterilization tray system in accordance with the first aspect of the present invention comprises a rigid, rectangularly shaped tray 20 having a pair of side walls 22, 24, a pair of end walls 26, 28, and a bottom wall 30 defining a generally rectangular interior space. Preferably tray 20 is provided with integral feet 33 so that the tray bottom wall 30 is spaced above the surface upon which it is placed.

Formed in the tray bottom wall 30 are a plurality of holes 32. Holes 32 are evenly spaced from one another by an on center distance D in a plurality of vertical, horizontal and diagonal columns. Holes 32 serve the dual purpose of permitting ingress or egress of steam or other sterilant to circulate through the tray, and also serve for locating instrument brackets or dividers as will be described in detail hereinafter, interiorly of the tray. In a preferred embodiment of the invention, holes 32 are formed in a cruciform shaped pattern having a central portion 34 and four evenly spaced lobes 36 (see Fig. 1a). However, the holes 32 may comprise a center portion 34 and three lobes 36 (see Fig. 1b), or a central portion 34 and two lobes 36 (Fig. 1c). In yet another embodiment of the invention, hole 32 comprises a dumb-bell shaped hole consisting of a large and a small lobe 36a, 36b, respectively.

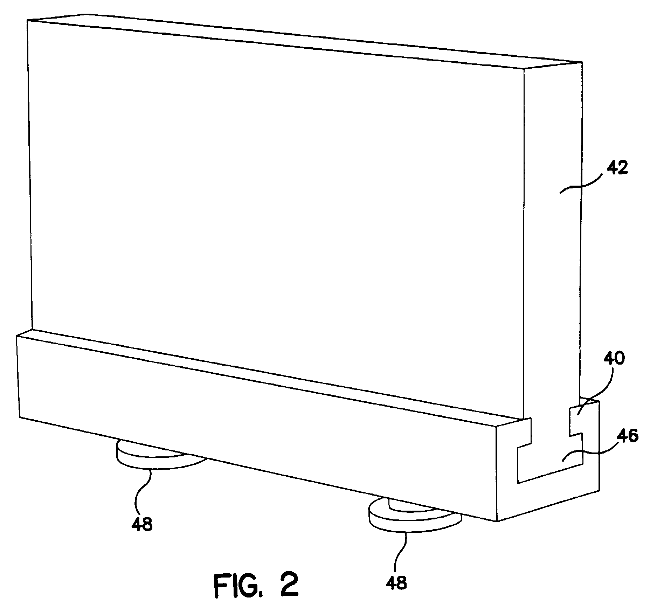

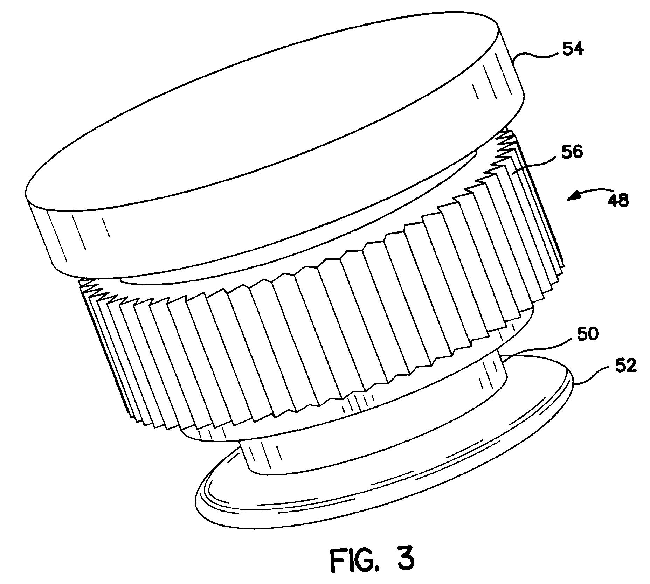

The sterilization tray system in accordance with the present invention also includes one or more support brackets 40 in which are mounted instrument dividers 42, or instrument clamps 44 or the like. Referring in particular to Figs. 2 and 3, bracket 42 comprises an elongated rail having a T-shaped channel 46 in which a selected divider 42 or instrument clamp 44 is slidably mounted. Each bracket 40, which may be formed of a rigid plastic or the like, is anchored in selected locations in holes 32 to the bottom wall 30 of the tray by means of posts 48 which extend through stepped holes 50 in brackets 40, and project from the bottom of the bracket. Each post 48 has a reduced diameter neck or groove 50 and a flange 52 formed at the distal end thereof, and an enlarged head 54 and a knurled body portion 56 for press-fitting into stepped holes 50 in bracket 40. Reduced diameter neck 50 has a length approximating that of the thickness of tray bottom wall 30.

Posts 48 are spaced apart by a distance equal to twice the distance between holes 32. Also, flange portions 52 are sized to fit through central portion 34 of holes 32 (shown in dotted lines), but are oversized relative to the node portions 36. Thus, posts 48 may be loaded into holes or removed from holes 32 by the center portions. However, the brackets 40 may then be locked in place by sliding the brackets with their associated posts into the nodes 36.

A feature and advantage of the present invention is the ability to universally mount dividers and brackets at essentially any location on the tray. Thus, by providing holes 32 evenly spaced across the tray bottom wall, brackets 40 may be mounted at any location running in a horizontal, vertical or diagonal direction. Moreover, no special tools are needed for mounting and unmounting the brackets, it being a simple matter to located the brackets in the desired holes, and then slide the brackets from central locations in the holes into a corresponding node position whereupon the pins and associated brackets will become friction held in position on the tray.

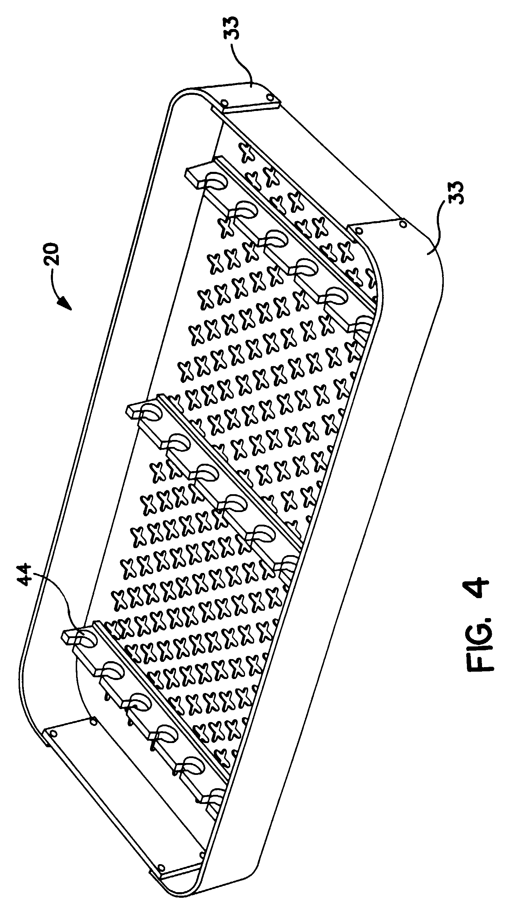

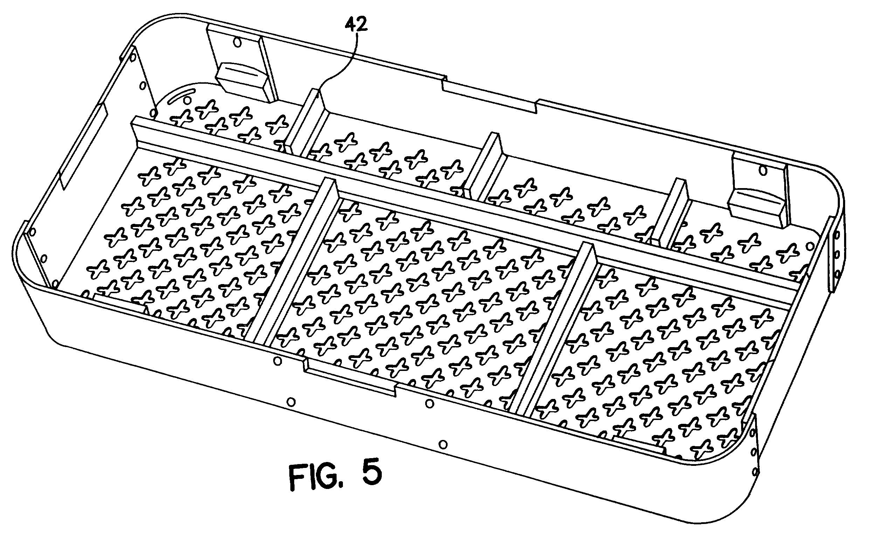

Referring to Figs. 4 and 5, in a preferred embodiment of the invention, brackets 40 comprise short stub brackets each having a pair of posts 48 extending therethrough. Of course, the brackets can be made longer, and have three or more spaced posts. However, an advantage of the present invention is that it permits the sterilization tray system to be manufactured, packaged and used as a kit of parts. Thus, a plurality of brackets 40 may be packaged together with a variety of dividers or slotted instrument clamps, etc., which may be made of, for example, silicone rubber. Silicone rubber is relatively easily cut. Thus, the user could purchase elongated strips or rolls of silicone rubber strips, cut them to desired length, and thus customize a tray in the field.

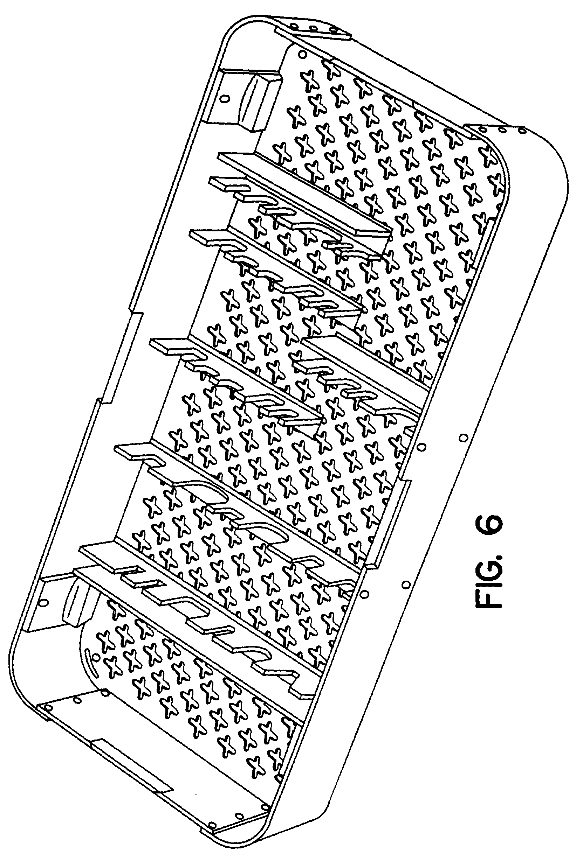

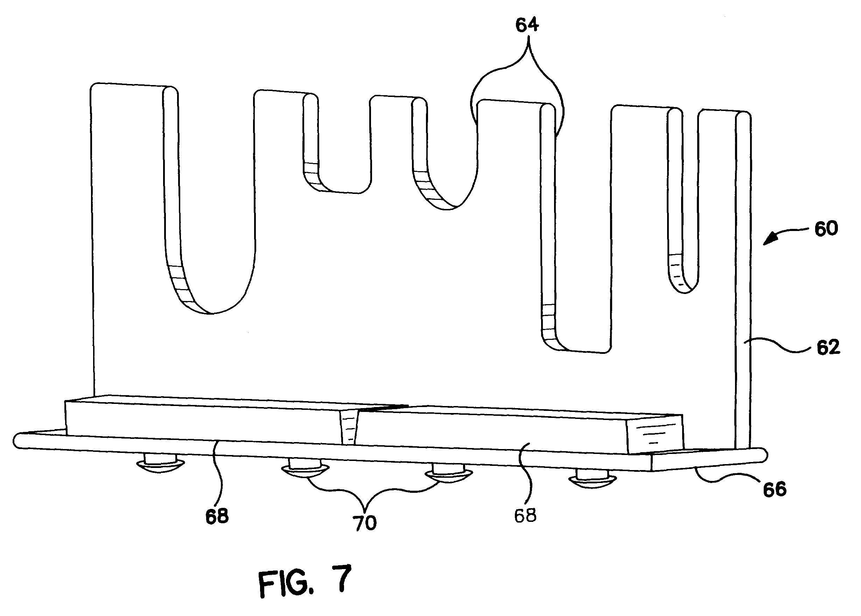

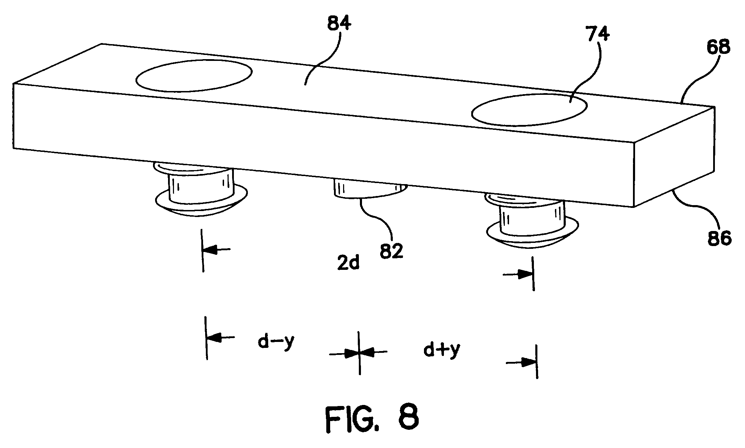

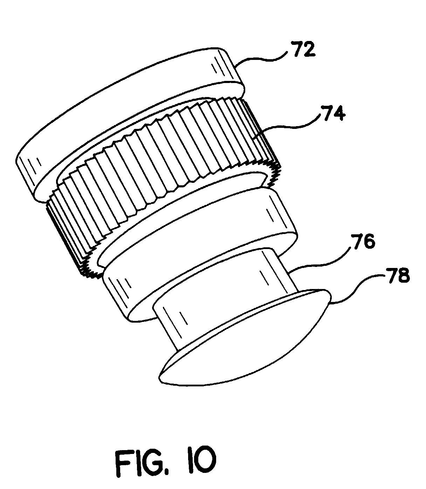

Figs. 6-11 illustrate alternative forms of posts, brackets, dividers, and instrument supports in accordance with the present invention. Referring in particular to Figs. 7-10, the instrument dividers/supports comprise generally L-shaped members 60 formed of silicone rubber or the like, and comprising a upright wall 62 in which may be provided one or more slots 64 for accommodating a surgical instrument (not shown) and a short base wall 66. Wall 66 is sandwiched between locking brackets 68, as will be described in detail hereinafter arid the tray bottom wall 30, by means of posts 70 which extend through holes (not seen) in walls 66 and walls 32 in the tray bottom wall 30. Referring in particular to Figs. 8, 9 and 10, post 70 which include an enlarged head 72 are mounted in holes 74 formed in bracket 68 and are friction held in place therein by knurled section 74. Each post also includes a reduced diameter neck 76 adjacent its distal end, inbound of a flared section 78. As before, posts 70 preferably are spaced apart a distance equal to twice the spacing D.

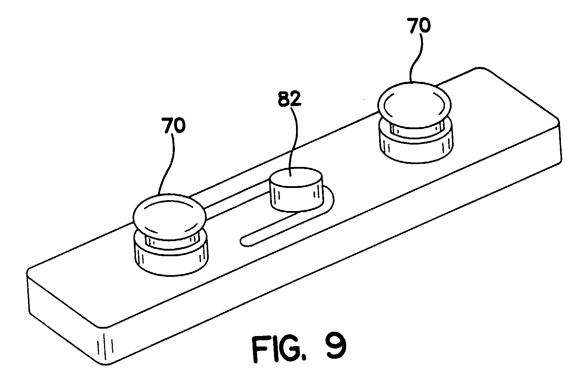

Referring in particular to Figs. 8 and 9, in a preferred embodiment of the invention bracket 64 includes a locking post 82 which is carried on a resiliently deformable arm 84. Locking post 82 normally extends below the lower surface 86 of bracket 68, and is located between posts 70, offset from the midpoint therebetween by a distance "Y" which equals the distance between hole central portions 34 and nodes 36. Thus, when brackets 68 are loaded in holes 32, and slid into a node position, locking post 82 snaps down locking the bracket in position. However, it is a simple matter to remove bracket 68 by pressing post 82 upwards, and then sliding the bracket back to the central position.

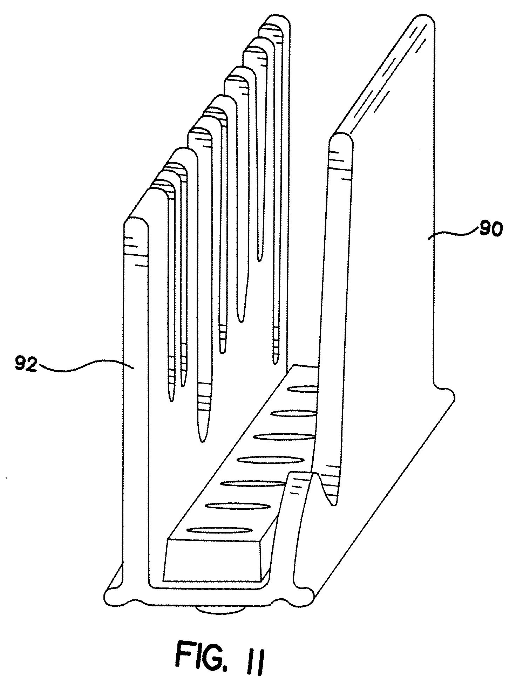

Various changes may be made in the invention without departing from the spirit and scope thereof. For example, as shown in Fig. 11, the instrument support may comprise a generally U-shaped member having a solid divider wall 90 and a slotted wall 92.

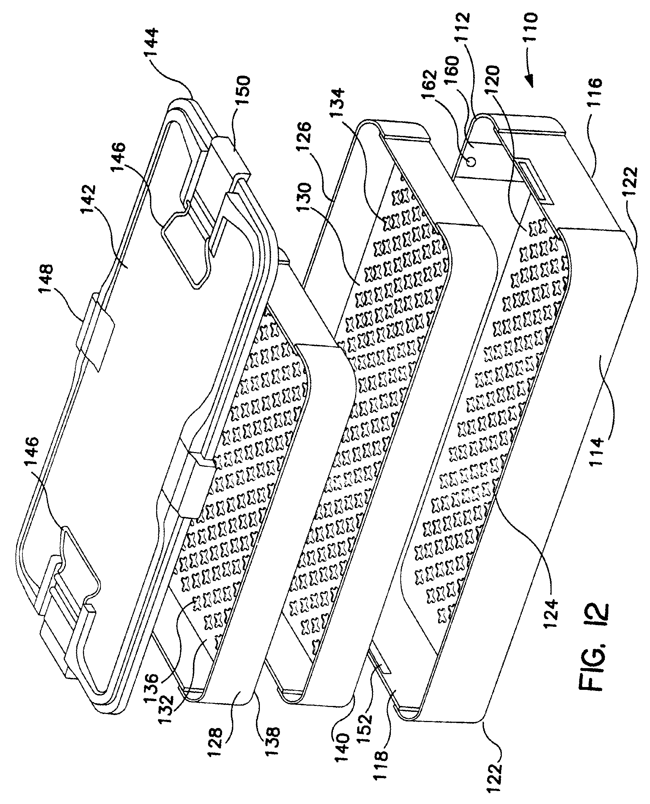

Referring to FIGS. 12-14 of the drawings, a tray system in accordance with a second aspect of the present invention comprises a rigid, generally box-like outer case 110 having a pair of side walls 112, 114, a pair of end walls 116, 118 and .a bottom wall 120 defining a generally rectangular interior space. Preferably, case 112 is provided with integral feet 122 so that the case bottom wall 120 is spaced above the surface upon which it is placed. Formed in the bottom wall 120 are a plurality of holes 124. Typically, holes 124 are evenly spaced from one another in a plurality of columns and rows. Holes 124 serve the dual purpose of permitting ingress and egress of steam or other sterilant to circulate throughout the case, and also may serve for locating instrument brackets and dividers 125 interiorly of the case. In a preferred embodiment of the invention, holes 124 are formed in a cruciform shaped patterns as will be described above.

The sterilization tray system in accordance with the present invention also includes one or more trays 126, 128 which are sized to fit within case 110. Just as in case 110, the bottom wall 130 of tray 126 and bottom wall 132 to tray 128 include columns and rows of cruciform shaped pattern holes 134, 136, respectively. Tray 126 is supported vertically above bottom wall 120 of case 110 by means of adjustable brackets as will be described in detail hereinafter. Tray 128 rests on top of tray 126. Tray 126 and tray 128 are both provided with integral legs 138 and 140, respectively. Legs 138 and 140 are located slightly inwardly, by the wall thickness of trays 126 and 128 so as to locate one tray stacked on top of the other.

The tray system also includes a removable top 142. Top 142 has a peripheral downwardly depending skirt 144 arranged to engage over the top rim of case 110. Top 142 includes a pair of lifting handles 146 and one or more pairs of locking hinges 148, 150 which may be pivotally or slidably mounted, in known manner, to top 142 for engaging suitably located slots or bosses 152 formed on or in case 110.

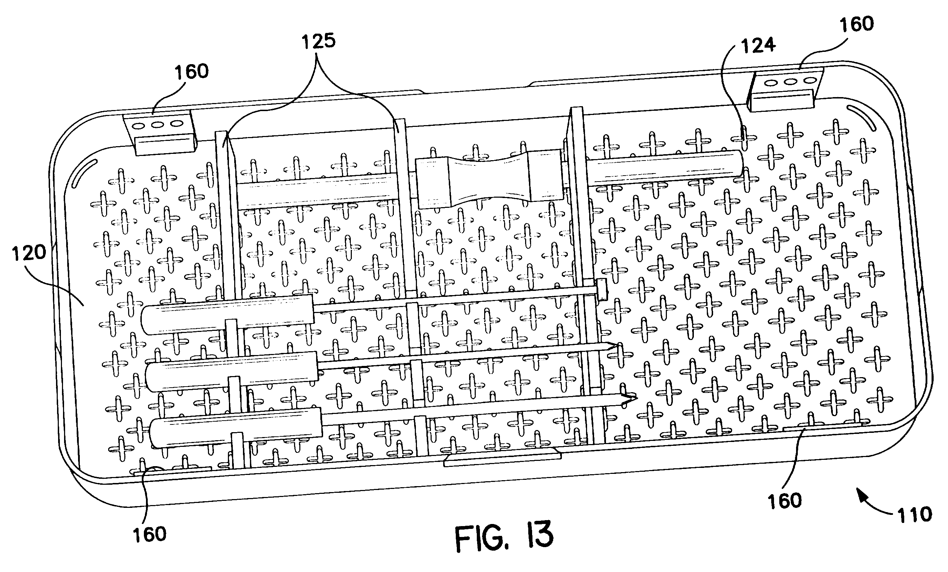

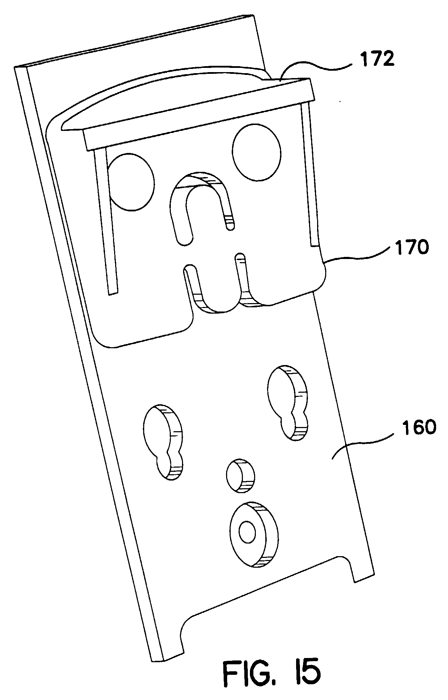

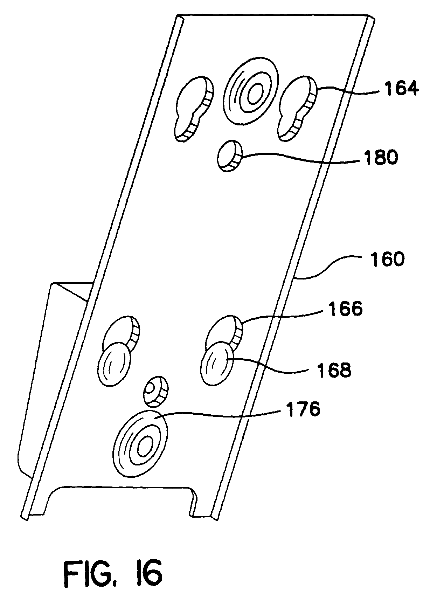

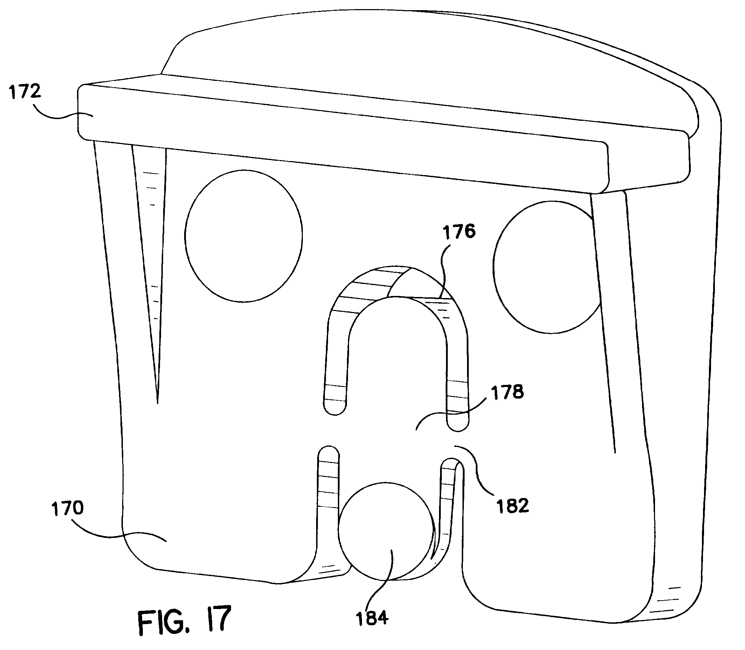

Referring also to FIGS. 15, 16 and 17, a feature and advantage of the present invention is to permit the stacking of one or more trays at selected vertical locations within the case. In order to accomplish this, a plurality of standards 160 are affixed to the case side and/or end walls of case 110. Preferably, but not necessarily, standards 160 are affixed in pairs opposite one another to the case side and/or end walls, i.e. as shown in FIG. 13. Standards 160 preferably are permanently affixed to the case side and/or end walls by means of mechanical fasteners such as rivets 162. Standards 160 include one or more vertically spaced locating holes 164, 166 for accommodating tabs or bosses 168 extending from the backside of shelf or tray brackets 170. In a preferred embodiment of the invention, standards 160 include two or more pairs of vertically spaced keyhole shaped holes 164, 166, while tabs or bosses 168 comprise button-shaped posts extending on reduced diameter stems from the back of each bracket 170. Bracket 170 also includes a shell 172 for engaging with slots 174 formed in the bottom walls of the trays.

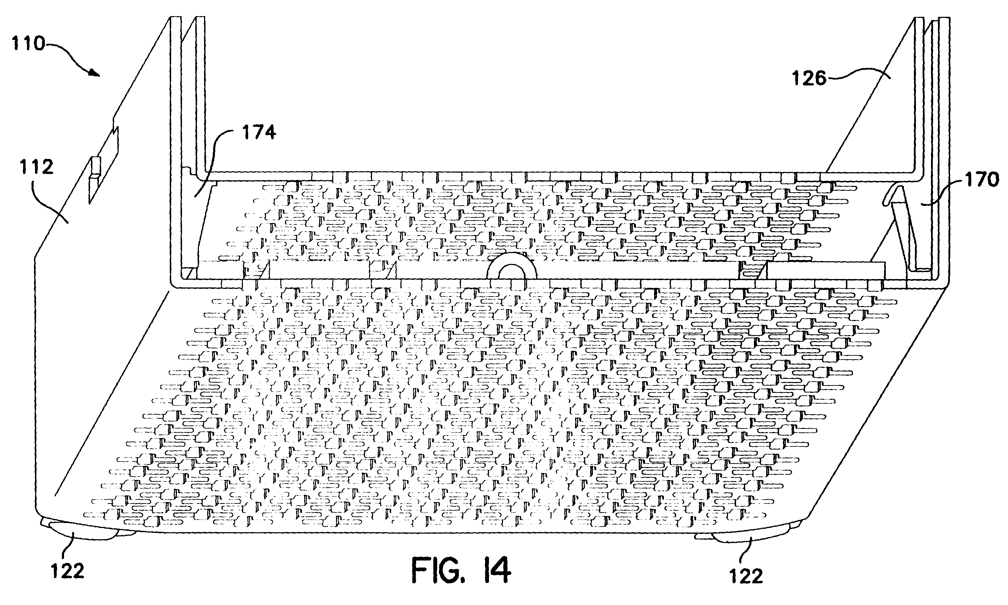

Preferably, and in order to lock brackets 170 on standards 160, brackets 170 include an engageable boss or tab 176 which is mounted on the backside of a pivotally mounted arm 178, for engaging in a hole 180 which preferably is located centrally and slightly below hole pairs 164 and 166. Arm 178 is integral with said bracket, and is made somewhat thinner than the main body of bracket 170, and is joined to the main body of bracket 170 by reduced thickness webs 182. Accordingly, by pressing down on the distal end 184 of arm 172, boss 176 may be pivotally lifted out of engagement with hole 180 in standard 160. This arrangement permits the user to change the vertical position of bracket 170 without using a tool, and without any loose parts. Thus, bracket 170 serves the dual purpose of permitting vertical adjustment of a tray within case 110, and also serves to locate and space a tray within case 110 spaced from the inner walls of the case (see Fig. 14), so as to permit circulation of steam or sterilant within the case, and also avoid trapping of condensation between the inner wall of case 110 and the outer wall of tray 126.

The invention is susceptible to modification. For example, four pairs of standards 160 may be affixed spaced along walls of case 110 for accommodating two trays each occupying substantially one-half the length of the case. Or, six pairs of standards 160 may be spaced for accommodating trays each occupying substantially one-third the length of the case, or one tray occupying approximately two-thirds the length and at tray occupying one-third the length. Alternatively, two or more pairs of standards 160 may be affixed to the end walls of the case for accommodating long, narrow width trays, occupying, for example, one half the width of the case (four standards), one third the width of the length (six standards) or a combination of trays including one occupying substantially two-thirds the width of the case and one occupying substantially one-third the width of the case.

Yet other possibilities are possible. For example, the standards may comprise elongate vertically mounted standards 190 including a plurality of horizontal slots 192 in which are mounted spring clips 194, i.e. similar to conventional bookcase shelf brackets.

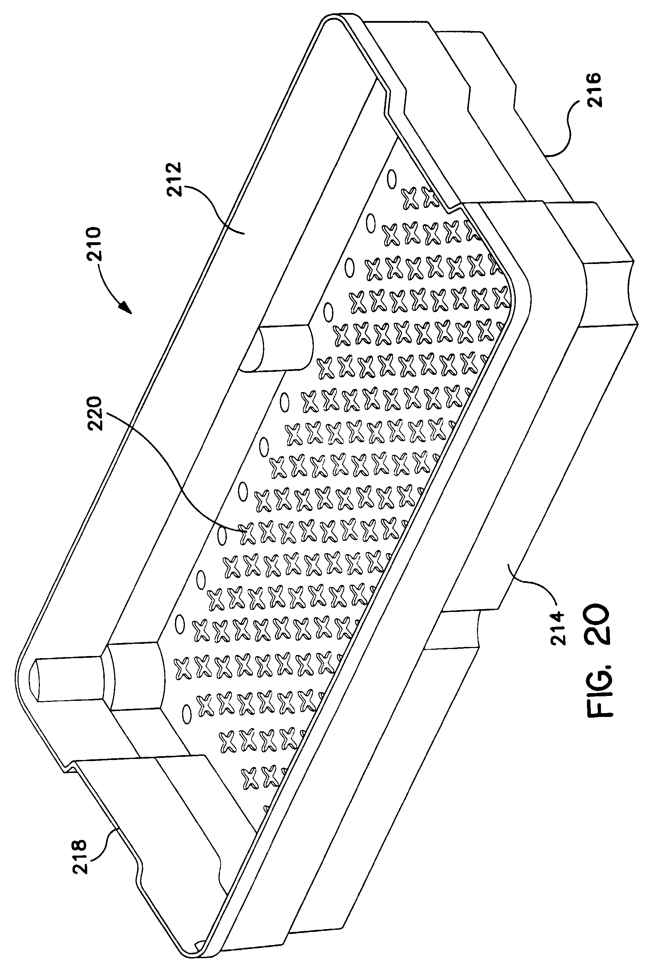

Referring to FIGS. 20-23 the drawings, the sterilization tray in accordance with the third aspect of the present invention comprises a pair of side walls 212, 214 and a pair of end walls 216, 218 defining a generally rectangular open frame 210. Side and end walls 212, 214, 216 and 218 comprise generally L-shaped members in cross section, and preferably are formed as a continuous frame element, e.g. by molding. However, the side and end walls may comprise extruded members joined together at the corners, for example, by mechanical fastening means or snap fittings, or by means of an adhesive or by means of plastic welding.

Mounted within the frame 210 and forming a base wall thereof is a metal plate 220. Metal plate 220 is formed of a rust-resistant material such as aluminum or stainless steel plate. A plurality of ventilation/mounting holes 222 are formed through plate 220, e.g. by stamping or drilling. Preferably holes 222 comprise cruciform shaped holes as described above.

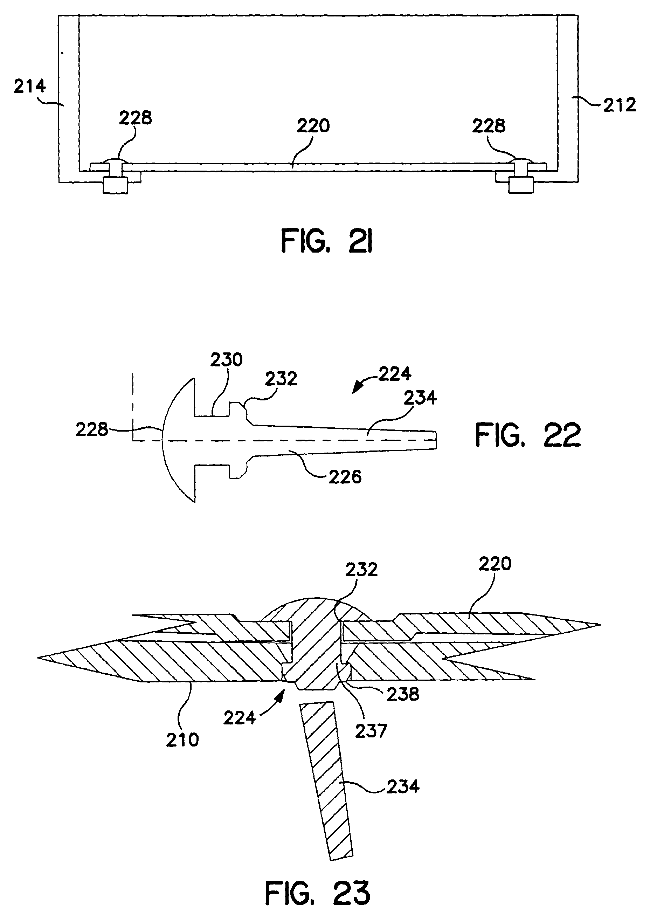

Referring in particular to Figs. 22 and 23, plate 220 is mounted in frame 210 by means of resilient mounting members which in a preferred embodiment comprises silicone rubber rivets 224. Silicon rubber rivets 224 each comprise an elongate body 226 including a flanged head 228, a reduced neck portion 230 and a retaining ring 232, and an elongated tail 234 which extends from retainer ring 232. Plate 220 is mounted in frame 210 by means of rivets 226 which extend through holes 232 formed in the peripheral edges of plate 220 in alignment with matching holes 236 formed in the frame 210. As can be seen in particular in Fig. 23, hole 232 is slightly oversized as compared to neck 230, while hole 236 includes step portion 237 and 238 which are sized so as to snugly capture the neck 230 and retaining ring portions 230 of rivets 224.

Assembly of plate 220 to frame 210 is quite straightforward. The plate is located in the frame with holes 232 aligned with holes 236. Then, rivets 224 are pressed and pulled through holes 232 and 236. Finally, tail portions 234 are cut off leaving rivets 224 more or less flush with the bottom of the tray.

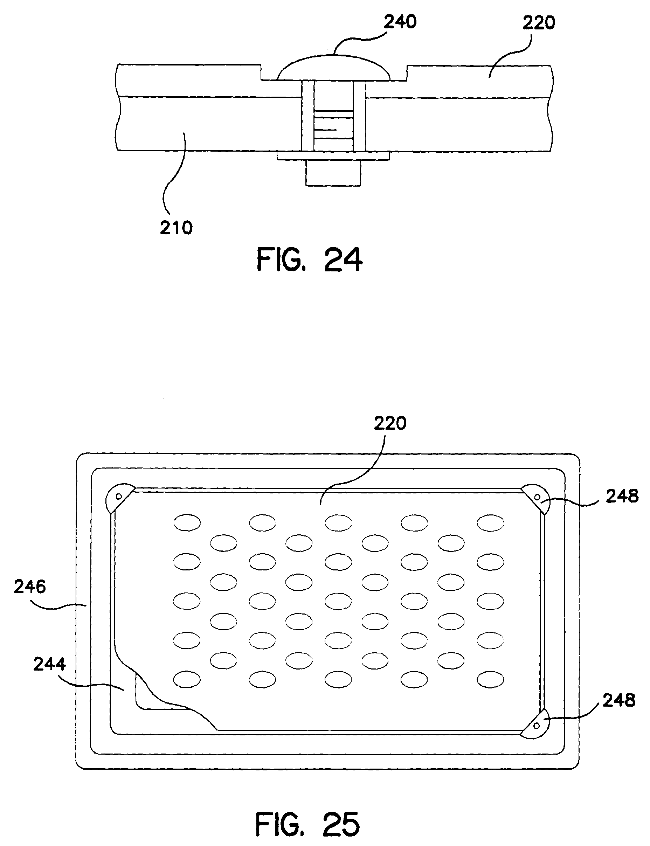

Rivets 224 are formed of resiliently deformable material compatible with sterilization conditions such as medical grade silicone rubber. Forming rivets 226 of medical grade silicone rubber has the advantage in that the silicone rubber is highly resiliently deformable and thus accommodates for differences in the coefficients of thermal expansion of metal plate 220 and plastic frame 210. It should be noted, however, that other means may be employed for mounting metal plate 220 in plastic frame 210. For example, as seen in Fig. 24, metal plate 220 may be mounted to frame 210 using other types of fasteners including, for example, interference fit fasteners, threaded fasteners, clips, etc., made of plastic, nylon or metal. For example, in the case of screw fasteners or the like, the fasteners 240 may have oversized heads and/or washers. It should be noted that where metal fasteners are used, the holes through the metal plate 220 and the plastic frame 210 should be made sufficiently oversized to accommodate anticipated differences in thermal expansion between the metal plate 220 and plastic frame 210.

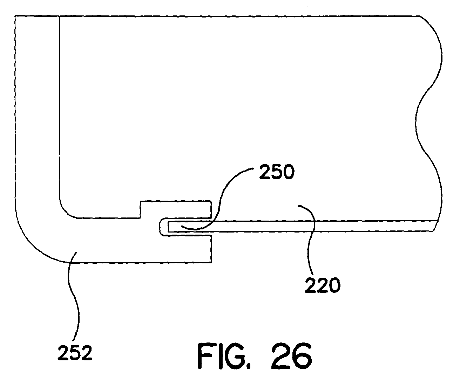

Fig. 25 illustrates yet another embodiment. In the Fig. 25 embodiment metal plate 220 is located in an L-shaped peripheral groove 244 formed in plastic frame 246, and is held in the plastic frame by corner brackets 248 which permit the metal plate to float in the frame. Referring to Fig. 26, in yet another embodiment, the metal plate 220 is captured in a channel 250 formed in the frame 252. In order to facilitate assembly, frame 252 may be formed in two or more pieces and joined together by means of suitable fasteners, snap fittings or adhesive.

## Claims

1. A combination including a sterilization tray assembly for sterilizing surgical instruments, said assembly including a tray having a plurality of holes and an upstanding element for clasping or separating surgical instruments on said tray, characterized in that the bottom wall 30 of said tray is populated at least in part with evenly spaced ventilation/mounting holes 32.

2. The combination of claim 1, characterized by one or more of the following features:
(a) wherein each of the ventilation/mounting holes 32 are identically sized and spaced;
(b) wherein the ventilation/mounting holes 32 each comprise a central portion 34 having at least one lobe 36 communicating therewith;
(c) wherein the ventilation/mounting holes 32 each comprise a central portion 34 having two lobes 36 communicating therewith;
(d) wherein the ventilation/mounting holes 32 each comprise a central portion 34 having three lobes 36 communicating therewith;
(e) wherein the ventilation/mounting holes 32 each comprise a central portion 34 having four lobes communicating therewith;
(f) wherein the ventilation/mounting holes 32 are cruciform-shaped; and
(g) wherein the ventilation/mounting holes 32 are evenly spaced from one another in rows, columns, and diagonals.

3. The combination of claim 1, characterized by further comprising a relatively rigid support element 40 removably mounted on said tray and supporting said upstanding element 42, 44.

4. The combination of 3, characterized by one of more of the following features:
(a) wherein said relatively rigid support element 40 comprises an elongated rail having a T-shaped channel 46 in which is slidably mounted said upstanding element 42, 44;
(b) wherein each relatively rigid support element 40 has integrally formed mounting posts 48 depending from the lower surface thereof, each post having a reduced diameter neck 50 and a flange 52 formed at a distal end thereof, the reduced diameter neck 50 having a length approximating that of the thickness of the bottom wall 30 of the tray; and
(c) wherein each relatively rigid support element 40 comprises two posts 48 spaced apart from one another by a distance 2D equal to twice the distance D between adjacent holes in the tray bottom 30;
(d) wherein the post flange portions 52 preferably are sized to fit through the holes' central portions 34, but are oversized relative to the hole nodes 36; and
(e) further comprising a locking post 82 extending below the lower surface of the rigid support element, between said mounting posts 70, said locking post 82 being located offset from the midpoint between said mounting posts by a distance Y equal to the distance between the hole central portions and the nodes.

5. The combination of claim 1, characterized by one or more of the following features:
(a) wherein said upstanding element 42, 44 comprises a resiliently deformable material, preferably silicon rubber;
(b) wherein said upstanding element comprises a generally L-shaped elongate member 60;
(c) wherein said upstanding element comprises a generally U-shaped elongate member;
(d) wherein said upstanding element comprises a generally U-shaped elongate member, wherein one leg 90 of the U comprises a solid wall, while the other leg 92 has one or more slots formed therein;
(e) wherein said upstanding element comprises a solid wall 42; and
(f) wherein said upstanding element includes at least one slot 64 formed therein.

6. A sterilization tray assembly comprising a rectangularly shaped tray having a bottom with ventilation/mounting holes formed therein, characterized in that the tray comprises a plastic frame 210 having floatably mounted therein a metal plate 220 which forms the bottom of the tray, the metal plate having ventilation holes/apertures 222 formed therein.

7. The sterilization tray of claim 6, characterized by one or more of the following features:
(a) wherein the metal plate 220 is formed of aluminum;
(b) wherein the metal plate 220 comprises stainless steel;
(c) wherein the metal plate 220 is affixed to the frame 210 by means of resiliently deformable fasteners 224;
(d) wherein the metal plate 220 is affixed to the frame 210 by means of rivets 224 formed of a resiliently deformable material, preferably silicone rubber;
(e) wherein the metal plate 220 is affixed 210 to the frame by means of fasteners 240 extending through oversized holes;
(f) wherein the metal plate 220 is supported in a groove 224 in the frame 246, and is held in the frame by brackets 248;
(g) wherein the metal plate 220 is supported in a channel 250 formed in the frame 252, and wherein the frame preferably is formed of at least two pieces which are joined together by means of a fastener such as snap fittings or an adhesive.

8. A modular sterilization tray system for medical instruments characterized by comprising in combination:
(a) a rigid, substantially rectangular case 110 having a pair of opposite side walls 112, 114, a pair of opposite end walls 116, 118 and a bottom wall 120 defining the generally rectangular interior space;
(b) pairs of standards 160 affixed vertically to opposite said side and/or end walls, said standards having a plurality of holes '64, 166 formed therethrough;
(c) at least one rectilinear tray for 126, 128 placement in said case; and
(d) brackets 170 for positioning in said holes in said standards for fixing the position of said one or more trays at selected elevations of said case.

9. The sterilization tray system of claim 1, characterized by further including a removable cover 142 for said case.

10. The sterilization tray system of claim 1, characterized by one or more of the following features:
(a) wherein said holes 164, 166 in said standards 160 are keyhole shaped;
(b) wherein said brackets 170 include a boss 176 for engaging a hole 180 in said standards 160;
(c) wherein said boss 176 is formed integrally with the bracket 170 and is joined to the main body of the bracket by reduced thickness webs 182;
(d) wherein said standards 190 include a plurality of slots 192 for accommodating removable spring clips 194;
(e) wherein the case bottom and tray bottom include a plurality of cruciform shaped holes 124 for accommodating removable instrument brackets and dividers 125;
(f) wherein said trays 126, 128 have integral legs 138, 140 located inwardly by a wall thickness so as to locate one tray stacked on top of the other;
(g) wherein said trays include slots formed on the bottom walls for locating on said brackets 170; and
(h) wherein said pairs of standards 160 are affixed opposite one another on opposite side and/or end walls.
